# EUROPÄISCHE PATENTANMELDUNG

(11) **EP 1 295 571 A1**
(43) Veröffentlichungstag der Anmeldung: **26.03.2003**
(21) Anmeldenummer: 02017509.7
(22) Anmeldetag: 06.08.2002
(51) Int. Cl.: A61F 2/06

(54) **Aorta-Implantat**

(30) Priorität: 25.09.2001 DE 20115706 U
(71) Anmelder: Curative AG, 01307 Dresden (DE)
(72) Erfinder: Friedrich, Holger, Dr., 01277 Dresden (DE); Chavan, Ajay, Dr., 30625 Hannover (DE); Haverich, Axel, Prof. Dr., 30916 Isernhagen (DE)
(74) Vertreter: Kailuweit, Frank, Dr. Dipl.-Ing.

(57) **Zusammenfassung**

Die Erfindung betrifft eine Anordnung zum Implantieren in eine Aorta, vorzugsweise den Aortenbogen geeignet. Der Erfindung liegt die Aufgabe zugrunde, eine derartige Anordnung zu schaffen, mit der einfache und schnell eine Mitbehandlung der Aortadiszendenz ermöglicht. Ferner soll der Zeitaufwand für die herzferne Anastomose reduziert werden. Gelöst wird die Aufgabe durch eine Anordnung die aus einem Stent (2) aus einem selbst- oder ballonexpandierbaren Metallgittergewebe, dass auf einen Durchmesser größer als der der deszendenten Aorta aufweitbar ist und einer flexiblen Verkleidung aus einem gewebeersetzenden Material, wobei die Verkleidung diesen in Längsrichtung (X) zumindest bereichsweise bedeckt und wenigstens auf einer Seite über das Ende des Stent (2) hinausragend ein Schlauchstück (3) bildet, das als Gefäßprothese dient. Derartige implantierbare Anordnungen (1) ermöglichen insbesondere bei der Behandlung von Aortenbogenaneurysmata unter Einbeziehung der Kopfarterien kurze Eingriffszeiten.

## Beschreibung

Die Erfindung betrifft eine Anordnung zum Implantieren in eine Aorta. Sie ist vorzugsweise zum Implantieren in einen Aortenbogen geeignet. Die Erfindung dient der Behandlung von Aortenbogenaneurysmen und kann bei Eingriffen am offenen Brustkorb angewendet werden.

Aus der Praxis sind zur Behandlung von Gewebeschädigungen sogenannten Stenosen verschiedene Implantationsanordnungen bekannt. Eine der Möglichkeiten besteht beispielsweise in einem dauerhaft im Gefäß angeordneten Stent. Hierbei handelt es sich um einen Spreizkörper aus einem selbst- oder ballonexpandierbaren Metallgittergewebe. Es hat sich jedoch gezeigt, dass ein Gefäßstent nach mehreren Wochen von einer neuen Gefäßinnenwand überzogen ist, was bei engen Gefäßen zu einer weiteren Stenose (Reststenose) führen kann.

Die US 5,213,580 beschreibt einen Ballon-Katheter mit einem Überzug aus gewebeverträglichem Kunststoff zur Behandlung von Gefäßabschnitten. Diese Kunststoffversiegelung soll nach dem Entfernen des Ballonkatheters für ein gleichbleibendes Offenhalten des betreffenden Gefäßabschnittes dienen. Nachteilig ist hierbei, dass der Kunststoffüberzug nur schwer im Körpergefäß zu befestigen ist und darüber hinaus in der Regel eine den zu therapierenden Gewebebereich stark übersteigende Fläche damit versiegelt werden muss.

In der DE 197 31 834 A1 wird eine Implantationsvorrichtung zur Behandlung von im Bereich der Innenwand von Hohlorganen verletztem oder erkranktem Gewebe, insbesondere zur Behandlung einer Dissektion in Körpergefäßen beschrieben, mit der ein selbst- oder ballonexpandierbarer Stent in das Gefäß eingebracht und mit Befestigungsmitteln, zum Beispiel Stifte oder Klammern an der Gefäßwand fixiert wird. Mit dieser Vorrichtung soll eine streng lokale Behandlung von verletztem oder erkranktem Gewebe der Innenwand von Hohlorganen unter Reduzierung der Gewebebelastung auf ein Minimum ermöglicht werden. Die Gefäßprothese besteht aus einem gewebeverträglichem Material, z. B. poröser Kunststoff oder Nitinol. Bei einer solchen Anordnung ist es besonders nachteilig, dass durch das Einbringen der Befestigungsmittel die Dauer des Eingriffs bedenklich ist, so dass insbesondere bei der Verwendung im Aortabogen eine erhöhte Gefahr einer Hirnschädigung besteht.
Das Gebrauchsmuster DE 91 16 881 betrifft einen Stent mit einem Metallgittergerüst, wobei das Metallgittergerüst mit einer flexiblen Ummantelung aus einem gewebeverträglichem Material. Derartige Stents werden auch als Stentgraft bezeichnet. Ein derartiger Stentgraft ist zur Behandlung von Aortenbogenaneurysmata unter Einbeziehung der Kopfkranzgefäße ungeeignet. Ein weiterer Nachteil ist darin zu sehen, dass bei Einsatz eines herkömmlichen Stentgraft bzw. einer gefäßersetzenden Gefäßprothese nicht auf die Präparation der Deszendenzaorta verzichtet werden kann, was mit einer längeren Operationszeit verbunden ist.

Der Erfindung liegt die Aufgabe zugrunde, eine Anordnung zum Implantieren in eine Aorta, insbesondere für den Aortabogen zu schaffen, die einfache und schnell eine Mitbehandlung der Aortadiszendenz ohne zusätzlichen Zeitaufwand oder gar eine zweite Operation ermöglicht. Ferner soll der Zeitaufwand für die herzferne Anastomose reduziert werden. Des weiteren soll sich die Anordnung als therapeutisches Mittel zur Behandlung von Aortenbogenaneurysmen und zur Verhinderung einer Ruptur eignen.

Diese Aufgabe wird mit einer Anordnung gemäß den im Patentanspruch 1 aufgeführten Merkmalen dadurch gelöst, dass die Anordnung zum Implantieren in eine Aorta, insbesondere in den Bereich des Aortabogens, einen Stent aus einem selbst- oder ballonexpandierbaren Metallgittergewebe, dass auf einen Durchmesser größer als der der deszendenten Aorta aufweitbar ist und eine flexible Verkleidung aus einem gewebeersetzenden Material umfasst, wobei die Verkleidung an der Innenund/oder Außenwand des Stent angebracht ist und diesen in Längsrichtung zumindest bereichsweise bedeckt und wenigstens auf einer Seite über das Ende des Stent hinausragend ein Schlauchstück bildet, das als Gefäßprothese dient.

Mit der Erfindung ist eine Anordnung geschaffen worden, mit der insbesondere Aortenbogenaneurysmen behandelt und nachfolgende Rupturen verhindert werden können. Die Anordnung lässt, da sie weder zusätzliche Befestigungsmittel noch einer präparierten Deszendenzaorta bedarf, sich schnell und relativ unkompliziert in das geschädigte Gefäß einsetzen. Vorteilhaft lässt sich die Anordnung zur Behandlung von Gefäßkrümmungen zum Beispiel am Aortabogen einsetzen. Dort kann bei beidseitigen Aneurysmen der Bereich der Kopfarterien mit einem einzigen Implantat, nämlich der erfindungsgemäßen Anordnung überbrückt werden. Das freie Ende des Schlauchstückes kann mit einer weiteren Prothese oder direkt mit dem Aortagewebe auf übliche Art und Weise verbunden werden.

Vorteilhafte Ausführungen der Erfindung sind in den Unteransprüche 2 bis 5 angegeben. So besteht die Verkleidung des Stent und das den Stent überragende Schlauchstück zweckmäßigerweise aus einem flexiblen gewebeverträglichen Kunststoff.

Um eine bessere Anpassung der implantierbaren Anordnung an die verschiedenen Anforderungen im Bereich des Aortenbogens und innerhalb der Deszendenzaorta zu erzielen, ist es günstig, dass die Verkleidung des Stent und das über den Stent hinausstehende Schlauchstück aus unterschiedlichen Materialien besteht.

Vorteilhaft besteht die Verkleidung des Stent aus Dacron und das überstehende Schlauchstück aus blutdichtem oder blutdicht beschichtetem Material.

Damit der Stent 2 mit der Verkleidung nicht ggf. in diesem Bereich befindliche Gefäßabgänge blockiert, ist es vorteilhaft, dass der Stent in Längsrichtung nur zu 20 bis 90% mit Verkleidungsmaterial bedeckt ist.

Die Erfindung soll anhand eines Ausführungsbeispiels und den Zeichnungen näher erläutert werden.

Es zeigen:
- Figur 1:: ein beidseitiges Aneurysma am Aortabogen,
- Figur 2:: eine schematische Darstellung einer erfindungsgemäßen implantierbaren Anordnung,
- Figur 3:: eine schematische Darstellung einer erfindungsgemäßen Anordnung in situ in einer Aorta,

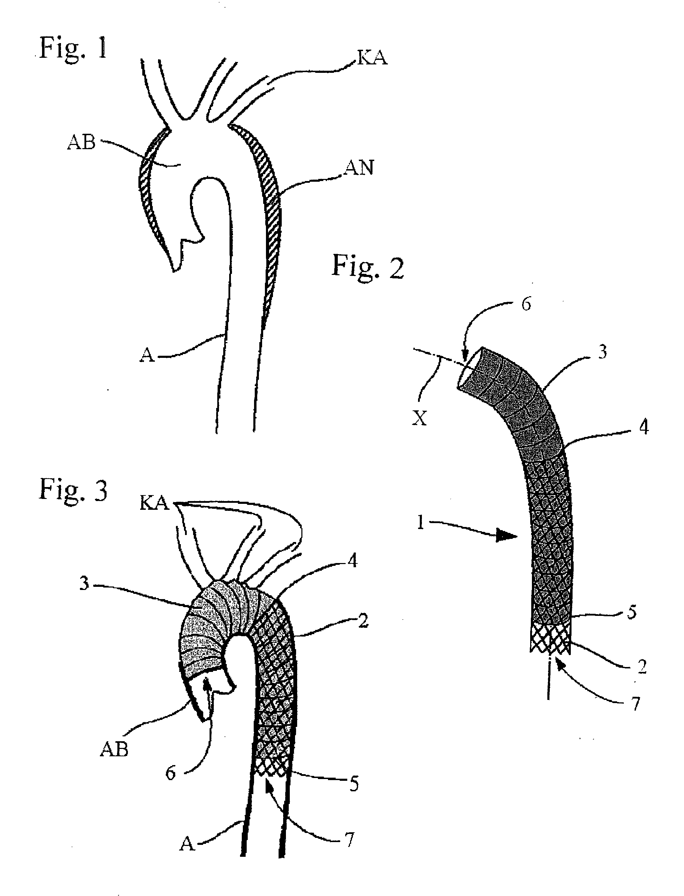

In Figur 1 ist eine Aorta dargestellt, bei der am Aortenbogen beidseitig der Kopfarterien KA Aneurysmen AN vorhanden sind.

Die in Fig. 2 dargestellte erfindungsgemäße implantierbare Anordnung 1 besteht aus einem Stent 2 der aus einem selbstexpandierbaren rohrförmigen Metallgittergewebe aus Nitinol besteht, welches im Bereich zwischen dem herznahen Ende 4 und dem herzfernen Ende 5 des Stents auf dessen Außenseite mit blutdichtem Dacron verkleidet ist. Mit der am herznahen Teil des Stents 2 befindlichen Verlängerung der Verkleidung aus dem gleichen Material ist das flexible Schlauchstück 3 ausgebildet, das als Gefäßprothese dient. Mit X ist die Längsachse der implantierbaren Anordnung 1 bezeichnet. Zur Verhinderung von Blockaden von der Aorta abzweigender Gefäße, ist die Verkleidung des Stent 2 zweckmäßigerweise nur auf 80 % der Längsrichtung X ausgebildet. Die konkreten Festlegungen für die Länge des Stent 2 und der Verkleidung sowie des freien Schlauchstückes 3 kann nur nach exakter Diagnose der Schädigung im Bereich des Aortenbogens festgelegt werden.

Zur Behandlung des Aortenbogenaneurysma bei offenem Brustkorb wird der Aortenbogen AB abgeschnitten und zwar direkt oberhalb der Aortenklappe und direkt hinter den sogenannten Kopfarterien KA. Das Aortengewebe wird dann verworfen. Die erfindungsgemäße implantierbare Anordnung wird mit einem geeigneten Implantationsbesteck in die Aorta deszendenz, in die herabsteigende Thoraxaorta mit dem von dem Stent 2 gebildeten herzfernen Ende 7 eingebracht und freigelassen, so dass sich der aus Nitinol bestehende Stent 2 dank seiner Memoryeigenschaft auf einen Durchmesser größer als die Aortainnenwand an diese deszendenz anlegen kann. Nunmehr ist der mit Gewebe von der Aorta A teilweise bedeckte Stent 2 in der Aorta deszendenz implantiert, so dass der Stent 2 mit seinem herzfernen Ende 7 einige Zentimeter unterhalb der Absatzstelle endet. Der Stent 2 braucht an seinem herzfernen Ende 7 keiner weiteren Befestigungsmittel. Nach oben hin hat der Chirurg dann das freie Schlauchstück 3 der implantierbaren Anordnung 1 zur Verfügung. Durch dieses Schlauchstück 3 ist es, wie in Figur 3 dargestellt, möglich, entsprechende Krümmungen und Freiräume zu überbrücken und den Aortenbogen AB zu ersetzen. Das herznahe Ende 6 des Schlauchstückes 3 kann dann mit dem Restgewebe der Aorta mittels einer Naht wieder verbunden werden. Des weiteren besteht die Möglichkeit, für die komplette Behandlung des Aortenbogenaneurysma eine zweite, weniger komplexe Gefäßprothese an dem herznahen Ende 6 des Schlauchstückes 3 in die Aorta einzubringen, die dann an das herznahe Ende 6 des Schlauchstücks 3 in üblicher Weise befestigt wird. Durch das Einbringen des selbstexpandierbaren Stent 2 in die Deszendenzaorta, der aufgrund Überdehnung und Friktion fest mit dem Gewebe verankert ist, wird eine große Naht und damit weitere Operationszeit eingespart. Diese Zeitersparnis hilft das Leben des Patienten nicht zu gefährden. Die Patienten müssen bei solch einem Eingriff künstlich auf 17°C heruntergekühlt werden. Um einen Hirnschaden zu vermeiden, eine künstliche Zirkulation aber aus technischen Gründen nicht möglich ist, sollte die Operationszeit auf maximal 50 Minuten begrenzt sein.

### Bezugszeichenliste:

- 1: implantierbare Anordnung
- 2: Stent
- 3: Schlauchstück
- 4: herznahes Ende der Überdeckung
- 5: herzfernes Ende der Überdeckung
- 6: herznahes Ende des Schlauchstücks 3
- 7: herzfernes Ende des Stent 2
- X: Längsachse
- A: Aorta
- AB: Aortenbogen
- AN: Aneurysma
- KA: Kopfarterien

## Patentansprüche

1. Anordnung zum Implantieren in eine Aorta (A), insbesondere in den Bereich des Aortenbogens (AB), umfassend einen Stent (2) aus einem selbst- oder ballonexpandierbaren Metallgittergewebe, dass auf einen Durchmesser größer als der der deszendenten Aorta (A) aufweitbar ist und einer flexiblen Verkleidung aus einem gewebeersetzenden Material, wobei die Verkleidung an der Innen- und/oder Außenwand des Stent (2) angebracht ist und diesen in Längsrichtung (X) zumindest bereichsweise überdeckt und wenigstens auf einer Seite über das Ende (4) des Stent (2) hinausragend ein Schlauchstück (3) bildet, das als Gefäßprothese dient.

2. Anordnung nach Patentanspruch 1, **dadurch gekennzeichnet, dass** die flexible Verkleidung aus einem gewebeverträglichen Kunststoff besteht.

3. Anordnung nach Patentanspruch 1 oder 2, **dadurch gekennzeichnet, dass** die flexible Verkleidung des Stent (2) und das über den Stent (2) überstehende Schlauchstück (3) aus unterschiedlichen Materialien bestehen.

4. Anordnung nach Patentanspruch 3, **dadurch gekennzeichnet, dass** das die Verkleidung aus Dacron und das überstehende Schlauchstück aus blutdichtem oder blutdicht beschichtetem Material besteht.

5. Anordnung nach einem der Ansprüche 1 bis 4, **dadurch gekennzeichnet, dass** die Verkleidung den Stent (2) von herznahen Ende (4) aus in Längsrichtung (X) zu 20 bis 90% bedeckt.
